(19) Europäisches Patentamt
European Patent Office
Office européen des brevets

(11) Publication number: **0 394 553 B1**

(12) **EUROPEAN PATENT SPECIFICATION**

(45) Date of publication of patent specification: **21.06.95**

(51) Int. Cl.[6]: **A61K 31/47**, A61K 31/495, A61K 31/44

(21) Application number: **89120396.0**

(22) Date of filing: **03.11.89**

The file contains technical information submitted after the application was filed and not included in this specification

# (54) Use of fluorine containing pyridone carboxylic acid derivatives for preparing a medicament for the treatment of HIV infections.

(30) Priority: **28.04.89 JP 110800/89**
**24.07.89 JP 191062/89**

(43) Date of publication of application:
**31.10.90 Bulletin 90/44**

(45) Publication of the grant of the patent:
**21.06.95 Bulletin 95/25**

(84) Designated Contracting States:
**AT BE CH DE ES FR GB GR IT LI NL SE**

(56) References cited:
**EP-A- 0 165 474**
**WO-A-90/13542**

**J. GEN. VIROL., vol. 64, part 10, 1983, pages 2329-2333; Y. SUMIYOSHI et al.: "Inhibition of retrovirus RNA-dependent DNA polymerase by novobiocin and nalidixic acid"**

**INN. MED., vol. 12, no. 3, 1985, pages 139-146; F. GUTZLER et al.: "Gyrase inhibitors - a new group of antimicrobial agents"**

(73) Proprietor: **DAIICHI PHARMACEUTICAL CO., LTD.**
**14-10, Nihonbashi 3-chome**
**Chuo-ku,**
**Tokyo 103 (JP)**

(72) Inventor: **Ohta, Genkichi**
**Daiichi Pharm. Res. Lab.**
**16-13, Kitakasai 1-chome**
**Edogawa-ku**
**Tokyo (JP)**
Inventor: **Furusawa, Mitsuru**
**Daiichi Pharm. Res. Lab.**
**16-13, Kitakasai 1-chome**
**Edogawa-ku**
**Tokyo (JP)**
Inventor: **Nozaki, Junko**
**15-6-306, Shinjuku 6-chome**
**Shinjuku-ku**
**Tokyo (JP)**
Inventor: **Iino, Takashi**
**Daiichi Pharm. Res. Lab.**
**16-13, Kitakasai 1-chome**
**Edogawa-ku**
**Tokyo (JP)**

**EP 0 394 553 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

ONCOLOGY, vol. 45, 1988, pages 107-116, S. Karger AG, Basel, DE; W. WALDECK et al.: "Topoisomerase II inhibitors influence simian virus 40 chromatin structure in vivo accompanied with inhibition of replication, transcription and changes in DNA supercoiling"

BIOCHEMICAL AND BIOPHYSICAL RESEARCH COMMUNICATIONS, vol. 121, no. 3, 29th June 1984, pages 762-769, Academic Press, Inc.; R. RUSOUET et al.: "Ouinolone antibiotics inhibit eucaryotic DNA polymerase alpha and beta terminal deoxynucleotidyl transferase but not DNA ligase"

ANTIMICROBIAL AGENTS AND CHEMOTHERAPY, vol. 31, no. 5, May 1987, pages 774-779, American Society for Microbiology; A. FORSGREN et al.: "Effects of ciprofloxacin on eucaryotic pyrimidine nucleotide biosynthesis and cell growth"

IMMUNOPHARMACOLOGY, vol. 13, no. 2, April 1987, pages 99-109, Elsevier; Y. ROCHE et al.. "Effects of quinolones on interleukin 1 production in vitro by human monocytes"

ANTIVIRAL RESEARCH, vol. 8, no. 3, 1987, pages 103-113, Elsevier; S. IKEDA et al.: "Antiviral activity and inhibition of topoisomerase by ofloxacin, a new quinolone derivative"

PROC. NATL. ACAD. SCI. USA, vol. 84, February 1987, pages 950-954, Biochemistry; L. YANG et al.: "Roles of DNA topoisomerases in simian virus 40 DNA replication in vitro"

THE JOURNAL OF ANTIBIOTICS, vol. 42, no. 1, January 1989, pages 107-115; Y. TAKE et al.: "Comparative studies of the inhibitory properties of antibiotics on human immunodeficiency virus and avian myeloblastosis virus reverse transcriptases and cellular DNA polymerases"

ANTIVIRAL CHEMISTRY, vol. 1, no. 2, 1990, pages 131-138; G. TACHEDJIAN et al.: "Investigation of topoisomerase inhibitors for activity against human immunodeficiency virus: inhibition by coumermycin A1"

(74) Representative: **Kinzebach, Werner, Dr. et al**
**Patentanwälte**
**Reitstötter, Kinzebach und Partner**
**Postfach 86 06 49**
**D-81633 München (DE)**

## Description

This invention relates to the use of fluorine-containing pyridone-carboxylic acid derivatives for preparing a pharmaceutical composition for treatment of HIV infection and diseases caused by HIV.

It is known that human immunodeficiency virus (HIV) is causative of acquired immunodeficiency syndrome, the so-called AIDS, in humans infected therewith. As a result of investigations in search of anti-HIV agents, several effective agents have already been developed. Neither of them is fully satisfactory, however.

Thus, even azidothymidine (AZT), which is said to be a highly effective chemotherapeutic agent, cannot be free from problems; long-term administration of AZT will cause adverse reactions due to bone marrow suppression, such as anemia and neutropenia, and allow development of resistance to AZT in HIV (see, N. Engl. J. Med., vol. 317, pp. 185-191 (1987) and N. Engl. J. Med., vol. 317, pp. 192-197 (1987)).

J. Gen. Virol. Vol. 64, 2329-2333 (1988) describes investigations with novobiocin and nalidixic acid with respect to their effect on the activity of RNA-dependent DNA polymerases from murine and avian retroviruses. It was shown that with isolated virus particles the viral DNA synthesis is inhibited by nalidixic acid (which is known as gyrase inhibitor, see for example Inn. Med. 12, 139-146 (1985)) and that this inhibition is due to the effect of this drug on RNA-dependent DNA polymerase.

It has now been found that various fluorine-containing pyridonecarboxylic acid derivatives can inhibit the growth of HIV in cells infected therewith on one hand and, on the other, promote cell proliferation. Antiviral activity of ofloxacin and the like against vaccinia virus and herpes virus was disclosed in JP-A-60-202822 (the term "JP-A" as used herein means an "unexamined published Japanese patent application").

The present invention therefore relates to the use of a fluorine-containing pyridonecarboxlic acid derivative of the general formula:

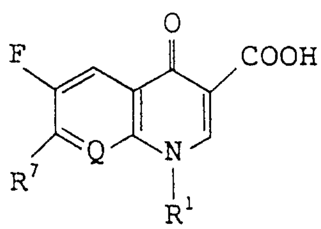

wherein Q is =N-, =C($R^8$)- ($R^8$ being H, F, Cl, an alkyl group having from one to four carbon atoms, preferably methyl, an alkoxyl group having from one to four carbon atoms, preferably methoxyl, or, $-OCH_2CH(R^{81})-$, $-SCH_2CH(R^{81})-$, $-OCH_2N(R^{81})-$ ($R^{81}$ being H or an alkyl group having from one to four carbon atoms, preferably methyl), the latter three groups being connected with the pyridone nitrogen atom to complete an additional ring such as an 1,4-oxazine ring whereby $R^1$ is not present; $R^1$ is a $C_1$-$C_8$ alkyl group, a cyclopropyl group, a halocyclopropyl group (e.g., cis-fluorocyclopropyl), a haloethyl group, a vinyl group, a phenyl group or a halophenyl group (e.g., 2-fluorophenyl, 4-fluorophenyl or 2,4-difluorophenyl); and $R^7$ is a nitrogen-containing heterocyclic group (e.g., piperazinyl, pyrrolidinyl, piperidinyl) of the formulae:

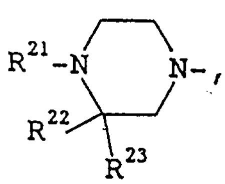 , 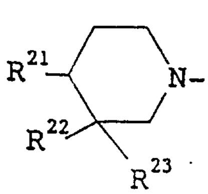 or 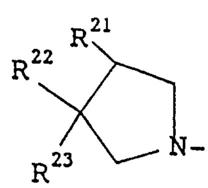

wherein $R^{21}$, $R^{22}$ and $R^{23}$ each independently is a hydrogen atom, a halogen atom, an amino group, a $C_1$-$C_8$ alkyl group, a $C_1$-$C_8$ alkoxy group or an amino $C_1$-$C_8$ alkyl group, and two of them may be combined with each other to form a spiro ring,

for preparing a pharmaceutical composition for treatment of human immunodeficiency virus infection and diseases caused by human immunodeficiency virus.

As specific examples of the nitrogen-containing heterocyclic group, there may be mentioned, among others, 3-aminopyrrolidinyl, 3-methylaminopyrrolidinyl, 3-dimethylaminopyrrolidinyl, 3-ethylaminopyrrolidinyl, 3-propylaminopyrrolidinyl, 3-isopropylaminopyrrolidinyl, 3-amino-4-methylpyrrolidinyl, 3-amino-5-methylpyrrolidinyl, 3-amino-4,5-dimethylpyrrolidinyl, 3-methylamino-4-methylpyrrolidinyl, 3-methylamino-5-methylpyrrolidinyl, 3-methylamino-4,5-dimethylpyrrolidinyl, 3-dimethylamino-4-methylpyrrolidinyl, 3-dimethylamino-5-methylpyrrolidinyl, 3-dimethylamino-4,5-dimethylpyrrolidinyl, 3-methylpiperazinyl, 4-methylpiperazinyl, 3,4-dimethylpiperazinyl, 3,5-dimethylpiperzinyl, 3,4,5-trimethylpiperazinyl, 4-ethyl-3,5-dimethylpiperazinyl, 4-isopropyl-3,5-dimethylpiperzinyl, 3-aminomethylpyrrolidinyl, 3-methylaminomethylpyrrolidinyl, 3-(1-amino)-ethylpyrrolidinyl, 3-(1-methylamino)ethylpyrrolidinyl, 3-(1-ethylamino)ethylpyrrolidinyl, 3-(1-amino)-propylpyrrolidinyl, 3-(1-methylamino)propylpyrrolidinyl, 3-amino-4,4-dimethylpyrrolidinyl, 7-amino-5-azaspiro[2,4]heptane-5-yl, 8-amino-6-azaspiro[3,4]octane-6-yl, 3,4-diazabicyclo[3,2,1]octane-2-yl, 3,9-diazabicyclo[3,2,1]octane-3-yl, 9-methyl-3,9-diazabicyclo[3,2,1]octane-3-yl and 9-ethyl-3,9-diazabicyclo-[3,2,1]octane-3-yl.

The fluorine-containing pyridonecarboxylic acid thus includes, within the scope thereof, quinolinecarboxylic acid compounds, naphthyridine carboxylic acid compounds and pyridobenzoxazine carboxylic acid compounds. These compounds are disclosed, for example, in JP-A-53-141286, JP-A-58-74667, JP-A-59-67279, JP-A-60-64979, JP-A-60-174786 and JP-A-60-228479, Japanese Patent Application Nos. 1-106762, 1-123366 and 1-156316, EP-A-0 206 283, U.S. Patent 4,382,892, and U.S. Patent Application Serial Nos. 06/876,623, 07/345,567 and 07/400,835, and can be produced by the methods disclosed in the respective patent specifications.

Among such compounds, those pyridonecarboxylic acid derivatives which have so far been developed are known under the designation of new quinolone compounds having excellent antimicrobial activity [cf. Naika (Internal Medicine), 62 (1), 28-33 (1988) and Farumashia, 25 (5), 434-440 (1989)]. Antimicrobial compounds of the same type which are to be developed in the future, namely further compounds of the above general formula, which have the 6-fluoro-quinoline-3-carboxylic acid, 6-fluoro-1,8-naphthyridine-3-carboxylic acid or 9-fluoro-2,3-dihydro-7-oxo-7H-pyrido-[1,2,3-de][1,4]benzoxazine-6-carboxylic acid skeleton with one or more appropriate substituents, will show excellent anti-HIV activity as well. Such compounds may also be used in the form of salts, or hydrates. Specific examples of such compounds include (-)-(S)-9-fluoro-2,3-dihydro-3-methyl-10-(4-methyl-1-piperazinyl)-7-oxo-7H-pyrido[1,2,3-de][1,4]-benzoxazine-6-carboxylic acid hemihydrate (levofloxacin, DR-3355), 1-ethyl-6,8-difluoro-7-(3-methyl-1-piperazinyl)-1,4-dihydro-4-oxoquinoline-3-carboxylic acid hydrochloride (lomefloxacin), 1-(4-fluorophenyl)-6-fluoro-7-(4-methyl-1-piperazinyl)-1,4-dihydro-4-oxo-quinoline-3-carboxylic acid hydrochloride (difluoxacin), 1-(2,4-difluorophenyl)-6-fluoro-7-(3-methyl-1-piperazinyl)-1,4-dihydro-4-oxo-quinolinino-3-carboxylic acid hydrochloride (A-62254), 1-(2-fluoroethyl)-6,8-difluoro-7-(4-methyl-1-piperazinyl)-1,4-dihydro-4-oxo-quinoline-3-carboxylic acid (fleroxacine), 1-(2,4-difluorophenyl-6-fluoro-(3-amino-1-pyrrolidinyl)-1,4-dihydro-4-oxo-1,8-naphthyridine-3-carboxylic acid methanesulfonate (T-3262), 1-cyclopropyl-6-fluoro-7-(2,6-dimethyl-4-pyridyl)-1,4-dihydro-4-oxo-quinoline-3-carboxylic acid (WIN57273), 5-amino-1-cyclopropyl-6,8-difluoro-7-(3,5-dimethyl-1-piperazinyl)-1,4-dihydro-4-oxo-quinoline-3-carboxylic acid (AT-4140), (S)-9-fluoro-2,3-dihydro-3-methyl-10-(2,6-dimethyl-4-pyridyl)-7-oxo-7H-pyrido[1,2,3-de][1,4]-benzoxazine-6-carboxylic acid, (S)-9-fluoro-2,3-dihydro-3-methyl-10-(4-pyridyl)-7-oxo-7H-pyrido[1,2,3-de][1,4]-benzoxazine-6-carboxylic acid (CP-92121), 1-epoxymethano-7-fluoro-8-(4-methyl-1-piperazinyl)5-oxo-5H-thiazolo[3,2-a]quinoline-4-carboxylic acid hydrochloride (KB-5246), or 6-fluoro-1-methyl-7-(1-piperazinyl )-4-oxo-4H-[1,3]thiazedo-[3,2-a]-quinoline-3-carboxylic acid (NAD-394).

Those which are on the market and can be used in the practice of the invention include ofloxacin [OFLX; (±)-9-fluoro-3-methyl-10-(4-methyl-1-piperazinyl)-7-oxo-2,3-dihydro-7H-pyrido[1,2,3-de][1,4]-benzoxazine-6-carboxylic acid] and the 3S form thereof, norfloxacin [NFLX; 1-ethyl-6-fluoro-1,4-dihydro-4-oxo-7-(1-piperazinyl)-3-quinoline-carboxylic acid], ciprofloxacin hydrochloride [CPFX; 1-cyclopropyl-6-fluoro-1,4-dihydro-4-oxo-7-(1-piperazinyl)-3-quinoline carboxylic acid hydrochloride hydrate] and enoxacin [ENX; 1-ethyl-6-fluoro-1,4-dihydro-4-oxo-7-(1-piperazinyl)-1,8-naphthyridine-3-carboxylic acid sesquihydrate], among others.

The compounds used according to the invention can be administered by such conventional methods as oral administration and intravenous drip. For oral administration, the composition may have the form of tablets, capsules, granules, powders, and syrups. For intravenous drip, the composition may have the form of solutions prepared by adding a solubilizing agent, such as an acid or alkali. For oral administration, an effective amount is from about 2 to about 200 mg/kg/day which may be devided two to four times per day, that is, suitable total daily dose for adult human lies within the range of about 100 to about 1,000 mg, in particular about 200 to about 600 mg. For intravenous injection, an effective amount is from about 1 to about 20 mg/kg/injection taken two times per day, that is, suitable injectable dose for adult human lies within the range of about 100 to about 600 mg taken two times per day, and in the case of a drip infusion, for example, 40 ml solution containing 200 mg is preferably administered.

The following examples are further illustrative of the invention.

Human T cell-derived, T4 antigen-positive CEM cells (provided by the Institut Pasteur, France) were used as host cells of HIV. Cell culture was carried out in a carbon dioxide-containing atmosphere in an incubator maintained at 37 °C according to a conventional method [cf. National Institute of Health (Japan) Alumni Association (ed.): [Virus Jikkengaku Soron (Experiments in Virology)", pages 131-151]. RPMI 1640 medium (Gibco) containing 10% fetal calf serum (FCS) was used as the cell culture medium. The culture supernatant ($5 \times 10^6$ $TCID_{50}$/ml) obtained by subjecting CEM cells to HIV (LAV 1) infection and stored at -70 °C was used as the HIV source. The abbreviation "$TCID_{50}$" means "50% tissue culture infectious dose" (cf. Hisao Uetake (ed.): "Virus-gaku (Virology)", Rikogakusha, Tokyo, page 472) and "MOI" means "multiplicity of infection" (cf. the monograph cited just above, page 36).

Example 1

Anti-HIV activity

The medium for cell culture (10 ml) placed in a plastic culture flask (25 $cm^2$) was inoculated with CEM cells at a cell density of $2.5 \times 10^5$ cells/ml. After 3 days of incubation, the culture was transferred to a 15-ml centrifuge tube and subjected to centrifugation at 1,000 revolutions per minute for 5 minutes for sedimentation of cells and removal of the culture medium.

The stock viral suspension was added to the host cells thus prepared to an MOI of 0.05 and the mixture was maintained at 37 °C for 90 minutes with stirring at 30-minute intervals for viral adsorption. The medium was added to HIV-infected cells to a cell density of $5.0 \times 10^5$ cells/ml to give an HIV-infected cell suspension.

A mixture (1.0 ml) of the three constituents mentioned below was added to each well of a 24-well plastic culture plate and the test substance final concentration was adjusted to 10 mcg/ml, 5 mcg/ml, 2.5 mcg/ml or 1 mcg/ml. The "no addition" wells (0 mcg/ml) served as controls.

| | |
|---|---|
| HIV-infected cell suspension | 0.5 ml |
| Medium (RPMI 1640, 10% FCS) | 0.4 ml |
| Physiological saline solution of the test substance | 0.1 ml |
| Total | 1.0 ml |

After 4 days of incubation following viral infection, the cells in each well on the 24-well culture plate were suspended uniformly by pipetting and a 0.5-ml portion of the suspension was separated. A 2.0-ml portion of test substance-free fresh medium was added to the remaining 0.5-ml portion of the cell suspension, followed by incubation. (For ofloxacin and DR-3355, continued addition experiments were also performed.)

Seven days after viral infection, pipetting was performed to give a uniform cell suspension in each well and a 0.5-ml portion of the suspension was separated.

Incubation was continued and, 10 days after viral infection, a uniform cell suspension was produced in the same manner in each well and a 1.5-ml portion thereof was separated. A fresh 2.0-ml portion of the medium free of (or containing) the test substance was added to the well, followed by further incubation.

Fifteen days after viral infection, the cells in each well were suspended uniformly in the same manner in the medium and a 2.0-ml portion of the suspension was separated. A fresh 2.0-ml portion of the medium free of (or containing) the test substance was added to the remaining 0.5 ml of the cell suspension and incubation was continued.

Medium exchange was performed in the same manner 20 days and 25 days after viral infection.

The cell suspensions collected 4 days, 7 days, 10 days, 20 days and 30 days after viral infection were tested for viable cell count and viable cell percentage by trypan blue staining. At the same time, viral antigen-positive cell percentage determination was carried out by immunofluorescence using an anti-HIV antiserum isolated from a healthy carrier of HIV.

The results thus obtained are shown below in Tables 1-1 to 1-5. The numerical values given each is the mean for the respective two wells on the culture plate. The viable cell count is the total cell count minus the count of dead cells stained with trypan blue. The viable cell percentage is the percentage of viable cells among all the cells including dead cells. The viral antigen-positive cell percentage is the percentage of cells indicating antigen expression as found among all the cells including dead cells.

Example 2

Cytotoxicity

The cell culture medium (10 ml) placed in a plastic culture flask (25 $cm^2$) was inoculated with CEM cells to a cell density of $2.5\times10^5$ cells/ml and incubation was performed for 3 days. Then, the culture was transferred to a 15-ml plastic centrifuge tube and centrifuged at 1,000 revolutions per minute for 5 minutes for medium removal. A fresh portion of the medium was added to the cells, the cell density being adjusted to $5.0\times10^5$ cells/ml. The following mixture (1.0 ml) containing the cell suspension prepared in the above manner was incubated in a 24-well plastic culture plate for 4 days:

| | |
|---|---|
| Cell suspension ($5.0\times10^5$ cells/ml) | 0.5 ml |
| Medium (RPMI 1640, 10% FCS) | 0.4 ml |
| Physiological saline solution of the test substance | 0.1 ml |
| Total | 1.0 ml |

The cells in each well were then suspended uniformly in the medium by pipetting and a 0.5-ml portion of the suspension was separated.

A fresh 2-ml portion of the test substance-free medium was added to the remaining cell suspension (0.5 ml) and, after a further 3-day incubation, a cell suspension was produced in the same manner and a 0.5-ml portion thereof was removed.

The cell suspensions collected in the above manner were subjected to viable cell count and viable cell percentage determinations by trypan blue staining.

As a result, ofloxacin, ciprofloxacin, enoxacin, norfloxacin, DR-3355 and azidothymidine each showed no cytotoxicity at concentrations not exceeding 10 mcg/ml.

Example 3

| Dosage form example (capsules) | |
|---|---|
| Fluorine-containing pyridone-carboxylic acid | 100.0 mg |
| Corn starch | 23.0 mg |
| Carboxymethylcellulose (CMC) calcium | 22.5 mg |
| Hydroxypropylmethylcellulose | 3.0 mg |
| Magnesium stearate | 1.5 mg |
| | 150.0 mg |

Example 4

| Dosage form example (injectable solution) | |
|---|---|
| DR-3355 | 20 g |
| Sodium chloride | 6 g |
| Distilled water for injection to make | 1,000 g |

As mentioned above, the active ingredient compounds according to the invention caused, in the experimental system described in Example 1, the viral antigen-positive cell percentage to decrease with time, without any reproliferation, although their HIV inhibiting activity was slow and mild.

Azidothymldine inhibited the growth of HIV in the early stage but later allowed HIV to reproliferate with time. It may be said that the compounds to be used in accordance with the invention show anti-HIV activity via mechanisms of action which differ from the mechanisms of action of azidothymidine and are preferable as anti-HIV agents.

The compounds to be used in accordance with the invention have low toxicity. For example, the following $LD_{50}$ values determined in mice may be mentioned: 5,450 mg/kg (oral) for ofloxacin, not less than 4 g/kg (oral) for ciprofloxacin, enoxacin and norfloxacin, and not less than 200 mg/kg (intravenous) for DR-3355.

Table 1-1

| Test substance OFLX (mcg/ml) | Viable cell count ($\times 10^4$ cells/ml) | | | | | Viable cell percentage (%) | | | | | Viral antigen-positive cell percentage (%) | | | | |
|---|---|---|---|---|---|---|---|---|---|---|---|---|---|---|---|
| | after 4 days | after 7 days | after 10 days | after 20 days | after 30 days | after 4 days | after 7 days | after 10 days | after 20 days | after 30 days | after 4 days | after 7 days | after 10 days | after 20 days | after 30 days |
| 4-day addition | | | | | | | | | | | | | | | |
| 10 | 260 | 63 | 280 | 310 | 320 | 94 | 73 | 85 | 92 | 95 | 74 | 63 | 40 | 14 | 3 |
| 5 | 250 | 60 | 170 | 320 | 330 | 93 | 59 | 77 | 86 | 97 | 82 | 50 | 33 | 5 | 4 |
| 2.5 | 260 | 58 | 83 | 300 | 300 | 96 | 67 | 67 | 95 | 97 | 80 | 67 | 49 | 4 | 6 |
| 1 | 260 | 43 | 13 | 34 | 10 | 96 | 53 | 21 | 87 | 37 | 88 | 80 | 77 | 16 | UN |
| Continuous addition | | | | | | | | | | | | | | | |
| 10 | 280 | 55 | 130 | 290 | 390 | 95 | 66 | 87 | 92 | 95 | 78 | 72 | 43 | 9 | 6 |
| 5 | 340 | 58 | 160 | 300 | 290 | 98 | 64 | 87 | 95 | 94 | 75 | 76 | 40 | 11 | 2 |
| 2.5 | 260 | 59 | 110 | 220 | 360 | 94 | 64 | 81 | 94 | 97 | 83 | 62 | 38 | 17 | 10 |
| 1 | 300 | 39 | 46 | 15 | 11 | 94 | 55 | 42 | 75 | 33 | 82 | 78 | 85 | UN | UN |
| 0 | 300 | 46 | 36 | 2 | 0 | 95 | 59 | 45 | 5 | 0 | 78 | ≥90 | 87 | UN | UN |

UN: Undeterminable because of the presence of a lot of cell debris.

Table 1-2

| Test substance (mcg/ml) | Viable cell count ($\times 10^4$ cells/ml) | | | | | Viable cell percentage (%) | | | | | Viral antigen-positive cell percentage (%) | | | | |
|---|---|---|---|---|---|---|---|---|---|---|---|---|---|---|---|
| | after 4 days | after 7 days | after 10 days | after 20 days | after 30 days | after 4 days | after 7 days | after 10 days | after 20 days | after 30 days | after 4 days | after 7 days | after 10 days | after 20 days | after 30 days |
| IIIa 10 | 180 | 61 | 260 | 310 | 330 | 88 | 74 | 93 | 91 | 94 | 82 | 75 | 26 | 18 | 6 |
| 1 | 240 | 37 | 19 | 20 | 14 | 92 | 57 | 30 | 78 | 40 | 72 | 65 | 30 | 20 | UN |
| IIIb 10 | 190 | 62 | 240 | 320 | 300 | 89 | 70 | 83 | 92 | 94 | 80 | 50 | 40 | 13 | 4 |
| 1 | 220 | 53 | 19 | 25 | 14 | 89 | 75 | 27 | 81 | 36 | 88 | 62 | UN | UN | UN |
| IIIc 10 | 200 | 51 | 250 | 340 | 320 | 92 | 67 | 93 | 93 | 95 | 80 | 47 | 9 | 9 | 3 |
| 1 | 220 | 46 | 28 | 10 | 10 | 87 | 60 | 33 | 55 | 32 | 82 | 70 | UN | UN | UN |
| IIId 10 | 190 | 60 | 280 | 290 | 300 | 88 | 67 | 91 | 95 | 94 | 78 | 50 | 25 | 11 | 4 |
| 1 | 250 | 50 | 24 | 12 | 9 | 91 | 62 | 28 | 60 | 31 | 72 | 70 | UN | UN | UN |
| Control 0 | 270 | 47 | 20 | 7 | 0 | 92 | 55 | 31 | 9 | 0 | 81 | 80 | UN | UN | UN |

IIIa: (±)-9-Fluoro-3-methyl-10-(4-ethyl-1-piperazinyl)-7-oxo-2,3-dihydro-7H-pyrido[1,2,3-de][1,4]benzoxazine-6-carboxylic acid

IIIb: (±)-9-Fluoro-3-methyl-10-(4-n-propyl-1-piperazinyl)-7-oxo-2,3-dihydro-7H-pyrido[1,2,3-de][1,4]benzoxazine-6-carboxylic acid

IIIc: (±)-9-Fluoro-3-methyl-10-(4-n-butyl-1-piperazinyl)-7-oxo-2,3-dihydro-7H-pyrido[1,2,3-de][1,4]benzoxazine-6-carboxylic acid

IIId: (±)-9-Fluoro-3-methyl-10-(4-isopropyl-1-piperazinyl)-7-oxo-2,3-dihydro-7H-pyrido[1,2,3-de][1,4]benzoxazine-6-carboxylic acid

UN: Undeterminable because of the presence of a lot of cell debris.

Table 1-3

| Test substance DR-3355 (mcg/ml) | Viable cell count (x $10^4$ cells/ml) | | | | | Viable cell percentage (%) | | | | | Viral antigen-positive cell percentage (%) | | | | |
|---|---|---|---|---|---|---|---|---|---|---|---|---|---|---|---|
| | after 4 days | after 7 days | after 10 days | after 20 days | after 30 days | after 4 days | after 7 days | after 10 days | after 20 days | after 30 days | after 4 days | after 7 days | after 10 days | after 20 days | after 30 days |
| 4-day addition | | | | | | | | | | | | | | | |
| 10 | 170 | 50 | 280 | 230 | 320 | 92 | 68 | 94 | 85 | 92 | 88 | 57 | 31 | 19 | ≤1 |
| 5 | 190 | 78 | 320 | 190 | 360 | 91 | 76 | 95 | 87 | 92 | 90 | 59 | 33 | 36 | 2 |
| 2.5 | 190 | 76 | 280 | 230 | 130 | 94 | 80 | 92 | 87 | 83 | 88 | 53 | 44 | 30 | 10 |
| 1 | 220 | 71 | 54 | 3 | 68 | 95 | 70 | 61 | 20 | 98 | 91 | 61 | 56 | UN | 16 |
| Continuous addition | | | | | | | | | | | | | | | |
| 10 | 160 | 43 | 220 | 260 | 260 | 91 | 75 | 92 | 87 | 83 | 89 | 55 | 31 | 21 | 4 |
| 5 | 160 | 51 | 260 | 320 | 210 | 89 | 66 | 95 | 89 | 75 | 87 | 53 | 37 | 29 | 4 |
| 2.5 | 210 | 53 | 290 | 390 | 270 | 94 | 68 | 94 | 95 | 88 | 87 | 51 | 25 | 35 | 11 |
| 1 | 190 | 65 | 110 | 11 | 360 | 94 | 69 | 61 | 50 | 98 | 88 | 52 | 43 | UN | 15 |
| 0 | 180 | 48 | 27 | 4 | 0 | 92 | 66 | 38 | 4 | 0 | 89 | 75 | UN | UN | UN |

UN: Undeterminable because of the presence of a lot of cell debris.

Table 1-4

| Test substance NFLX (mcg/ml) | Viable cell count (x $10^4$ cells/ml) | | | | | Viable cell percentage (%) | | | | | Viral antigen-positive cell percentage (%) | | | | |
|---|---|---|---|---|---|---|---|---|---|---|---|---|---|---|---|
| | after 4 days | after 7 days | after 10 days | after 20 days | after 30 days | after 4 days | after 7 days | after 10 days | after 20 days | after 30 days | after 4 days | after 7 days | after 10 days | after 20 days | after 30 days |
| 10 | 180 | 60 | 250 | 370 | 400 | 92 | 71 | 91 | 97 | 94 | 92 | 63 | 50 | 4 | ≤1 |
| 5 | 230 | 84 | 86 | 28 | 330 | 94 | 67 | 61 | 81 | 95 | 83 | 65 | UN | UN | ≤1 |
| 2.5 | 240 | 59 | 40 | 2 | 70 | 94 | 67 | 47 | 33 | 96 | 93 | 55 | UN | UN | ≤1 |
| 1 | 230 | 52 | 26 | 0 | 0 | 94 | 59 | 30 | 0 | 0 | 88 | 75 | UN | UN | NT |
| 0 | 188 | 52 | 32 | 1 | 0 | 95 | 58 | 43 | 14 | 0 | 87 | 78 | UN | UN | NT |

NFLX: Norfloxacin
UN: Undeterminable because of the presence of a lot of cell debris.
NT: Not tested

Table 1-5

| Test substance (mcg/ml) | Viable cell count (x $10^4$ cells/ml) after 4 days | after 7 days | after 10 days | after 20 days | after 30 days | Viable cell percentage (%) after 4 days | after 7 days | after 10 days | after 20 days | after 30 days | Viral antigen-positive cell percentage (%) after 4 days | after 7 days | after 10 days | after 20 days | after 30 days |
|---|---|---|---|---|---|---|---|---|---|---|---|---|---|---|---|
| CPFX | | | | | | | | | | | | | | | |
| 10 | 180 | 65 | 250 | 300 | 330 | 91 | 77 | 92 | 96 | 95 | 88 | 57 | 27 | 10 | ≤1 |
| 5 | 220 | 76 | 310 | 280 | 320 | 97 | 75 | 92 | 95 | 92 | 92 | 67 | 29 | 4 | ≤1 |
| 2.5 | 200 | 98 | 320 | 220 | 360 | 95 | 77 | 85 | 95 | 97 | 87 | 60 | 31 | 3 | ≤1 |
| 1 | 220 | 86 | 52 | 8 | 33 | 84 | 73 | 53 | 53 | 92 | 83 | 62 | 37 | 33 | NT |
| ENX | | | | | | | | | | | | | | | |
| 10 | 140 | 52 | 150 | 178 | 379 | 97 | 61 | 91 | 96 | 94 | 88 | 68 | 30 | 5 | ≤1 |
| 5 | 210 | 58 | 78 | 11 | 310 | 98 | 60 | 62 | 11 | 98 | 93 | 68 | UN | UN | ≤1 |
| 2.5 | 240 | 67 | 30 | 1 | 2 | 95 | 68 | 35 | 13 | 67 | 90 | 65 | UN | UN | NT |
| 1 | 220 | 64 | 29 | 7 | 0 | 95 | 66 | 41 | 50 | 0 | 88 | 70 | UN | UN | NT |
| 0 | 194 | 66 | 32 | 1 | 0 | 93 | 68 | 42 | 17 | 0 | 88 | 73 | UN | UN | NT |

CPFX: Ciprofloxacin hydrochloride
ENX: Enoxacin
UN: Undeterminable because of the presence of a lot cell debris.
NT: Not tested

The term lower alkyl, alkoxy etc. preferably means groups having 1 to 8, in particular 1 to 6 and especially preferred 1 to 4 carbon atoms.

## Claims

**1.** The use of a fluorine-containing pyridone-carboxylic acid derivative of the formula:

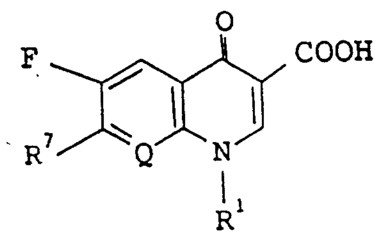

wherein Q is =N- or =C($R^8$)- in which $R^8$ is H, F, Cl, $C_1$-$C_4$ alkyl, $C_1$-$C_4$ alkoxy, -$OCH_2CH(R^{81})$-, -$SCH_2CH(R^{81})$- or -$OCH_2N(R^{81})$- the latter three groups being connected with the pyridone nitrogen atom to complete an additional ring whereby $R^1$ is not present and $R^{81}$ is H or $C_1$-$C_4$ alkyl; $R^1$ is $C_1$-$C_8$ alkyl, cyclopropyl, halocyclopropyl, haloethyl, vinyl, phenyl or halophenyl; and
$R^7$ is a group of the formula

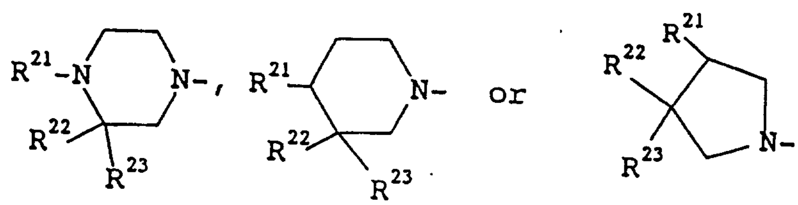

wherein $R^{21}$, $R^{22}$ and $R^{23}$ each independently is a hydrogen atom, a halogen atom, amino, $C_1$-$C_8$ alkyl, $C_1$-$C_8$ alkoxy or amino $C_1$-$C_8$ alkyl and two of them may be combined with each other to form a spiro ring; or a salt or hydrate thereof
for preparing a pharmaceutical composition for treatment of human immunodeficiency virus infection and diseases caused by human immunodeficiency virus.

**2.** The use according to claim 1, wherein said fluorine-containing pyridonecarboxylic acid derivative is a 3-quinolinecarboxylic acid with $R^1$ being cyclopropyl or halocyclopropyl and $R^8$ being H, F, Cl, $C_1$-$C_4$ alkyl or $C_1$-$C_4$ alkoxy.

**3.** The use according to claim 1, wherein said fluorine-containing pyridonecarboxylic acid derivative is a 1-$C_1$-$C_8$ alkyl-6-fluoro-1,4-dihydro-4-oxo-7-(1-piperazinyl- or $C_1$-$C_8$ alkyl-substituted 1-piperazinyl)-1,8-naphthyridine-3-carboxylic acid.

**4.** The use according to claim 1, wherein said fluorine-containing pyridonecarboxylic acid derivative is a 1-ethyl-6-fluoro-1,4-dihydro-4-oxo-7-(1-piperazinyl or $C_1$-$C_8$ alkyl-substituted 1-piperazinyl)-3-quinolinecarboxylic acid , with $R^8$ being H, F, Cl, $C_1$-$C_4$ alkyl or $C_1$-$C_4$ alkoxy.

**5.** The use according to claim 1, wherein said fluorine-containing pyridonecarboxylic acid derivative is a 9-fluoro-2,3-dihydro-3-methyl-10-(4-$C_1$-$C_8$ alkyl-1-piperazinyl)-7-oxo-7H-pyrido[1,2,3-de] [1,4]benzoxazine-6-carboxylic acid, a salt thereof or a hydrate thereof.

**6.** The use according to claim 5, wherein said fluorine-containing pyridonecarboxylic acid derivative has 3S configuration.

**7.** The use according to claim 1, wherein said fluorine-containing pyridonecarboxylic acid derivative is ofloxacin, the 3S form thereof (levofloxacin), ciprofloxacin, enoxacin, norfloxacin, lomefloxacin, fleroxacin, difloxacin, tosufloxacin (T-3262),
1-(2,4-difluorophenyl)-6-fluoro-7-(3-methyl-1-piperazinyl)-1,4-dihydro-4-oxo-quinolinino-3-carboxylic acid hydrochloride (A-62254) ,
5-amino-1-cyclopropyl-6,8-difluoro-7-(3,5-dimethyl-1-piperazinyl)-1,4-dihydro-4-oxo-quinoline-3-carboxylic acid (AT-4140), 1-cyclopropyl-6-fluoro-7-(2,6-dimethyl-4-pyridyl)-1,4-dihydro-4-oxo-quinoline-3-carboxylic acid (WIN57273), (S)-9-fluoro-2,3-dihydro-3-methyl-10-(4-pyridyl)-7-oxo-7H-pyrido[1,2,3-de][1,4]-benzoxazine-6-carboxylic acid (CP-92121), or (S)-9-fluoro-2,3-dihydro-3-methyl-10-(2,6-dimethyl-4-pyridyl)-7-oxo-7H-pyrido[1,2,3-de] [1,4]-benzoxazine-6-carboxylic acid.

8. The use according to claim 1, wherein said fluorine-containing pyridonecarboxylic acid derivative is 1-epoxymethano-7-fluoro-8-(4-methyl-1-piperazinyl)-5-oxo-5H-thiazolo[3,2-a]quinoline-4-carboxylic acid hydrochloride or 6-fluoro-1-methyl-7-(1-piperazinyl)-4-oxo-4H-[1,3]thiazedo[3,2-a]quinoline-3-carboxylic acid.

## Patentansprüche

1. Verwendung eines Fluor enthaltenden Pyridoncarbonsäurederivates der Formel:

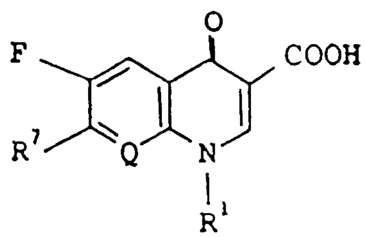

worin Q für =N- oder =C($R^8$)- steht, wobei $R^8$ für H, F, Cl, $C_1$-$C_4$-Alkyl, $C_1$-$C_4$-Alkoxy, -$OCH_2CH(R^{81})$-, -$SCH_2CH(R^{81})$- oder -$OCH_2N(R^{81})$- steht, wobei die letzten drei Gruppen mit dem Stickstoffatom des Pyridons verknüpft sind, um einen zusätzlichen Ring zu bilden, in dem $R^1$ nicht vorkommt und $R^{81}$ für H oder $C_1$-$C_4$-Alkyl steht; $R^1$ für $C_1$-$C_8$-Alkyl, Cyclopropyl, Halocyclopropyl, Haloethyl, Vinyl, Phenyl oder Halophenyl steht; und
$R^7$ eine Gruppe der Formeln

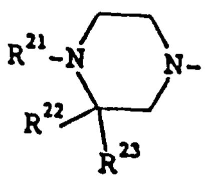,
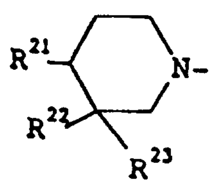
oder
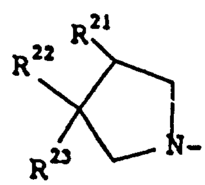

darstellt, worin $R^{21}$, $R^{22}$ und $R^{23}$ jeweils unabhängig ein Wasserstoffatom, Halogenatom, eine Amino-, $C_1$-$C_8$-Alkyl-, $C_1$-$C_8$-Alkoxy- oder Amino-$C_1$-$C_8$-Alkyl-Gruppe ist und zwei davon untereinander kombiniert sein können, um einen Spiroring zu bilden; oder eines Salzes oder Hydrates davon zur Herstellung eines pharmazeutischen Mittels für die Behandlung von Infektionen durch humanen Immunschwächevirus und Erkrankungen, hervorgerufen durch humanen Immunschwächevirus.

2. Verwendung gemäß Anspruch 1, worin dieses Fluor enthaltende Pyridoncarbonsäurederivat eine 3-Chinolincarbonsäure ist, wobei $R^1$ für Cyclopropyl oder Halocyclopropyl und $R^8$ für H, F, Cl, $C_1$-$C_4$-Alkyl oder $C_1$-$C_4$-Alkoxy steht.

3. Verwendung gemäß Anspruch 1, wobei dieses Fluor enthaltende Pyridoncarbonsäurederivat eine 1-$C_1$-$C_8$-Alkyl-6-fluor-1,4-dihydro-4-oxo-7-(1-piperazinyl- oder $C_1$-$C_8$-Alkyl-substituierte 1-Piperazinyl)-1,8-naphthyridin-3-carbonsäure ist.

4. Verwendung gemäß Anspruch 1, wobei dieses Fluor enthaltende Pyridoncarbonsäurederivat eine 1-Ethyl-6-fluor-1,4-dihydro-4-oxo-7-(1-piperazinyl oder $C_1$-$C_8$-Alkyl-substituierte 1-Piperazinyl)-3-chinolin-

carbonsäure ist, worin $R^8$ für H, F, Cl, $C_1$-$C_4$-Alkyl oder $C_1$-$C_4$-Alkoxy steht.

5. Verwendung gemäß Anspruch 1, wobei dieses Fluor enthaltende Pyridoncarbonsäurederivat eine 9-Fluor-2,3-dihydro-3-methyl-10-(4-$C_1$-$C_8$-Alkyl-1-piperazinyl)-7-oxo-7H-pyrido[1,2,3-de] [1,4]benzoxazin-6-carbonsäure ist, oder eines Salzes oder Hydrates davon.

6. Verwendung gemäß Anspruch 5, wobei dieses Fluor enthaltende Pyridoncarbonsäurederivat 3S-Konfiguration hat.

7. Verwendung gemäß Anspruch 1, wobei dieses Fluor enthaltende Pyridoncarbonsäurederivat Ofloxacin, die 3S-Form davon (Levofloxacin), Ciprofloxacin, Enoxacin, Norfloxacin, Lomefloxacin, Fleroxacin, Difloxacin, Tosufloxacin (T-3262), 1-(2,4-Difluorphenyl)-6-fluor-7-(3-methyl-1-piperazinyl)-1,4-dihydro-4-oxo-chinolino-3-carbonsäurehydrochlorid (A-62254), 5-Amino-1-cyclopropyl-6,8-difluor-7-(3,5-dimethyl-1-piperazinyl)-1,4-dihydro-4-oxo-chinolin-3-carbonsäure (AT-4140), 1-Cyclopropyl-6-fluor-7-(2,6-dimethyl-4-pyridyl)-1,4-dihydro-4-oxo-chinolin-3-carbonsäure (WIN57273), (S)-9-Fluor-2,3-dihydro-3-methyl-10-(4-pyridyl)-7-oxo-7H-pyrido[1,2,3-de][1,4]-benzoxazin-6-carbonsäure (CP-92121), oder (S)-9-Fluor-2,3-dihydro-3-methyl-10-(2,6-dimethyl-4-pyridyl)-7-oxo-7H-pyrido[1,2,3-de] [1,4]benzoxazin-6-carbonsäure ist.

8. Verwendung gemäß Anspruch 1, wobei dieses Fluor enthaltende Pyridoncarbonsäurederivat 1-Epoxymethan-7-fluor-8-(4-methyl-1-piperazinyl)-5-oxo-5H-thiazol[3,2-a]chinolin-4-carbonsäurehydrochlorid oder 6-Fluor-1-methyl-7-(1-piperazinyl)-4-oxo-4H-[1,3]thiazedo[3,2-a]chinolin-3-carbonsäure ist.

**Revendications**

1. Utilisation d'un dérivé d'acide pyridonecarboxylique fluoré de formule:

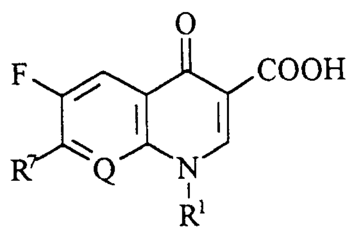

dans laquelle Q est =N- ou =C($R^8$)-, $R^8$ étant H, F, Cl, un groupe alkyle en $C_1$-$C_4$, un groupe alcoxy en $C_1$-$C_4$, -$OCH_2CH(R^{81})$-, -$SCH_2CH(R^{81})$- ou - $OCH_2N(R^{81})$-, les trois derniers groupes étant reliés à l'atome d'azote de la pyridone pour compléter un cycle supplémentaire, ce qui fait que $R^1$ n'existe pas, et $R^{81}$ est H ou un groupe alkyle en $C_1$-$C_4$; $R^1$ est un groupe alkyle en $C_1$-$C_8$, cyclopropyle, halogénocyclopropyle, halogénoéthyle, vinyle, phényle ou halogénophényle; et $R^7$ est un groupe ayant les formules:

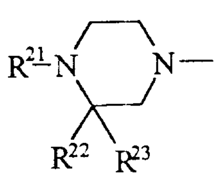 , 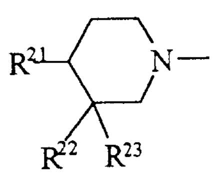 ou 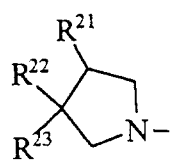

dans lesquelles $R^{21}$, $R^{22}$ et $R^{23}$ sont chacun indépendamment un atome d'hydrogène, un atome d'halogène ou un groupe amino, alkyle en $C_1$-$C_8$, alcoxy en $C_1$-$C_8$ ou aminoalkyle en $C_1$-$C_8$, et deux d'entre eux peuvent être combinés l'un avec l'autre pour former un noyau spiro; ou d'un de ses sels ou hydrates,

pour la préparation d'une composition pharmaceutique pour le traitement de l'infection par le virus du syndrome immunodéficitaire acquis et des maladies provoquées par le virus du syndrome immunodéficitaire acquis.

2. Utilisation selon la revendication 1, dans laquelle ledit dérivé d'acide pyridonecarboxylique fluoré est un acide 3-quinoléinecarboxylique, $R^1$ étant un groupe cyclopropyle ou halogénocyclopropyle et $R^8$ étant H, F, Cl, alkyle en $C_1$-$C_4$ ou alcoxy en $C_1$-$C_4$.

3. Utilisation selon la revendication 1, dans laquelle ledit dérivé d'acide pyridonecarboxylique fluoré est un acide 1-(alkyl en $C_1$-$C_8$)-6-fluoro-1,4-dihydro-4-oxo-7-(1-pipérazinyl-ou 1-pipérazinyl substitué par alkyle en $C_1$-$C_8$)-1,8-naphtyridine-3-carboxylique.

4. Utilisation selon la revendication 1, dans laquelle ledit dérivé d'acide pyridonecarboxylique fluoré est un acide 1-éthyl-6-fluoro-1,4-dihydro-4-oxo-7-(1-pipérazinyl- ou 1-pipérazinyl substitué par alkyle en $C_1$-$C_8$)-3-quinoléinecarboxylique, $R^8$ étant H, F, Cl, alkyle en $C_1$-$C_4$ ou alcoxy en $C_1$-$C_4$.

5. Utilisation selon la revendication 1, dans laquelle ledit dérivé d'acide pyridonecarboxylique fluoré est un acide 9-fluoro-2,3-dihydro-3-méthyl-10-[4-(alkyl en $C_1$-$C_8$)-1-pipérazinyl]-7-oxo-7H-pyrido[1,2,3-de][1,4]-benzoxazine-6-carboxylique, un de ses sels ou un de ses hydrates.

6. Utilisation selon la revendication 5, dans laquelle ledit dérivé d'acide pyridonecarboxylique fluoré a la configuration 3S.

7. Utilisation selon la revendication 1, dans laquelle ledit dérivé d'acide pyridonecarboxylique fluoré est l'ofloxacine, sa forme 3S (lévofloxacine), la ciprofloxacine, l'énoxacine, la norfloxacine, la loméfloxacine, la fléroxacine, la difloxacine, la tosufloxacine (T-3262), le chlorhydrate de l'acide 1-(2,4-difluoro-phényl)-6-fluoro-7-(3-méthyl-1-pipérazinyl)-1,4-dihydro-4-oxoquinoléine-3-carboxylique (A-62254), l'acide 5-amino-1-cyclopropyl-6,8-difluoro-7-(3,5-diméthyl-1-pipérazinyl)-1 ,4-dihydro-4-oxoquinoléine-3-carboxylique (AT-4140), l'acide 1-cyclopropyl-6-fluoro-7-(2,6-diméthyl-4-pyridyl)-1,4-dihydro-4-oxoquinoléine-3-carboxylique (WIN57273), l'acide (S)-9-fluoro-2,3-dihydro-3-méthyl-10-(4-pyridyl)-7-oxo-7H-pyrido[1,2,3-de][1,4]benzoxazine-6-carboxylique (CP-92121), ou l'acide (S)-9-fluoro-2,3-dihydro-3-méthyl-10-(2,6-diméthyl-4-pyridyl)-7-oxo-7H-pyrido[1,2,3-de][1,4]benzoxazine-6-c arboxylique.

8. Utilisation selon la revendication 1, dans laquelle ledit dérivé d'acide pyridonecarboxylique fluoré est le chlorhydrate de l'acide 1-époxyméthano-7-fluoro-8-(4-méthyl-1-pipérazinyl)-5-oxo-5H-thiazolo[3,2-a]-quinoléine-4-carboxylique ou l'acide 6-fluoro-1-méthyl-7-(1-pipérazinyl)-4-oxo-4H-[1,3]thiazédo[3,2-a]-quinoléine-3-carboxy lique.